# FASCICULE DE BREVET EUROPEEN

(11) **EP 3 407 902 B1**
(45) Date de publication et mention de la délivrance du brevet: **15.07.2020**
(21) Numéro de dépôt: 17702322.3
(22) Date de dépôt: 23.01.2017
(51) Int. Cl.: A61K 35/74, C12R 1/01, A23L 33/135, A61P 1/00, A61P 29/00

(54) **SOUCHE DE FAECALIBACTERIUM PRAUSNITZII CNCM 1-4573 POUR LE TRAITEMENT ET LA PREVENTION D'UNE INFLAMMATION GASTRO-INTESTINALE**
FAECALIBACTERIUM-PRAUSNITZII-STAMM CNCM 1-4573 ZUR BEHANDLUNG UND PRÄVENTION VON MAGEN-DARM-ENTZÜNDUNGEN
FAECALIBACTERIUM PRAUSNITZII STRAIN CNCM 1-4573 FOR THE TREATMENT AND PREVENTION OF GASTROINTESTINAL INFLAMMATION

(30) Priorité: 25.01.2016 FR 1650567
(43) Date de publication de la demande: 05.12.2018
(73) Titulaire: Institut national de recherche pour l'agriculture, l'alimentation et l'environnement, 75007 Paris (FR); Sorbonne Université, 75006 Paris (FR); Assistance Publique - Hôpitaux de Paris, 75004 Paris (FR)
(72) Inventeur: LANGELLA, Philippe, 78140 Velizy-Villacoublay (FR); MARTIN ROSIQUE, Rebeca, 78350 Jouy en Josas (FR); BERMUDEZ HUMARAN, Luis, 78350 Jouy en Josas (FR); CHAIN, Florian, 78000 Versailles (FR); SOKOL, Harry, 75015 Paris (FR)
(74) Mandataire: Nony
(86) Numéro de dépôt international: PCT/EP2017/051306
(87) Numéro de publication internationale: WO 2017/129515

(56) Documents cités:
- WO-A1-2016/075294
- MARTIN REBECA ET AL: "Faecalibacterium prausnitzii prevents physiological damages in a chronic low-grade inflammation murine model", BMC MICROBIOLOGY, vol. 15, mars 2015 (2015-03), XP002761622,
- MARTIN R ET AL: "The commensal bacterium faecalibacterium prausnitzii is protective in DNBS-induced chronic moderate and severe colitis models", INFLAMMATORY BOWEL DISEASES MARCH 2014 JOHN WILEY AND SONS INC. USA, vol. 20, no. 3, mars 2014 (2014-03), pages 417-430, XP009191583, ISSN: 1078-0998
- MINGMING ZHANG ET AL: "Faecalibacterium prausnitzii Inhibits Interleukin-17 to Ameliorate Colorectal Colitis in Rats", PLOS ONE, vol. 9, no. 10, 2 octobre 2014 (2014-10-02), page e109146, XP055186076, DOI: 10.1371/journal.pone.0109146
- Philippe Langella: "VHIR - Commensal Bacteria and Recombinant Lactic Acid Bacteria as Novel Probiotics for Human Intestinal Health", , 3 December 2012 (2012-12-03), page 1, XP055612277, Retrieved from the Internet: URL:http://en.vhir.org/portal1/seminari.as p?t=commensal-bacteria-and-recombinant-lac tic-acid-bacteria-as-novel-probiotics-for- human-intestinal-health&contentid=89829&s= activitats [retrieved on 2019-08-12]
- Philippe Langella: "Commensal Bacteria and Recombinant Lactic Acid Bacteria as Novel Probiotics for Human Intestinal Health Àrea General HUVH", , 3 December 2012 (2012-12-03), pages 1-1, XP055612279, Retrieved from the Internet: URL:http://en.vhir.org/DDGRecerca/DOCENCIA ACTIVITATS/2012/2012_0201/2012_0201_004.pd f [retrieved on 2019-08-12]
- Anonymous: "VHIR - Seminaris VHIR", , 1 January 2012 (2012-01-01), pages 1-4, XP055612282, Retrieved from the Internet: URL:http://www.vhir.org/portal1/seminaris. asp?s=activitats&anyo=2012&contentid=1264 [retrieved on 2019-08-12]

## Description

### Domaine de l'invention

La présente invention concerne une souche de *Faecalibacterium prausnitzii* pour son utilisation dans le traitement et la prévention de maladies gastro-intestinales inflammatoires (IBD) chez un individu, en particulier d'affections intestinales inflammatoires (IBD).

### Art antérieur

L'inflammation est un processus biologique naturel, qui constitue une partie normale de la réponse à des lésions ou des infections et contribue à la protection de l'organisme contre les agressions internes ou externes.

Cependant, un dysfonctionnement des mécanismes de l'inflammation, en particulier une inflammation persistante ou trop abondante, peut causer des maladies douloureuses et mettant en danger la vie du patient. De telles maladies comprennent, par exemple, des troubles cutanés, des troubles intestinaux, des troubles neurologiques, l'arthrite et des maladies auto-immunes. Plusieurs de ces maladies inflammatoires restent sans traitement ou sans traitement adéquat.

Par conséquent, l'étude et la recherche de nouvelles stratégies de traitement anti-inflammatoire constituent un sujet majeur en médecine et en recherche biomédicale.

Une affection intestinale inflammatoire est un groupe de troubles caractérisés par une inflammation chronique et récurrente du tractus gastro-intestinal. La forme la plus courante de ce groupe est la maladie de Crohn. La pathogenèse met en œuvre une activation inappropriée et continue du système immunitaire muqueux entraînée par la présence du microbiote intestinal chez un patient génétiquement prédisposé.

Il existe un besoin constant de nouvelles substances, en particulier de probiotiques, ou de compositions pour le traitement et/ou la prévention de maladies gastro-intestinales inflammatoires, en particulier des affections intestinales inflammatoires, chez un individu.

### Résumé de l'invention

L'objectif de la présente invention est de décrire de nouvelles substances, en particulier des probiotiques, et des compositions pour le traitement et/ou la prévention de maladies gastro-intestinales inflammatoires chez un individu, en particulier des affections intestinales inflammatoires chez un individu.

Dans le contexte de la présente invention, le terme « prévenir » désigne la réduction à un degré moindre du risque ou de la probabilité d'occurrence d'un phénomène donné, c'est-à-dire, dans la présente invention, une inflammation gastro-intestinale, en particulier une inflammation intestinale.

La présente invention est basée sur la découverte par les présents inventeurs des propriétés anti-inflammatoires de la souche de *Faecalibacterium prausnitzii* déposée auprès de la CNCM sous le numéro d'accession CNCM I-4573.

Selon les résultats expérimentaux des inventeurs, une souche spécifique de *Faecalibacterium prausnitzii* (*F. prausnitzii*) déposée auprès de la CNCM le 21 janvier 2012 sous le numéro d'accession CNCM I-4573 possède la capacité inattendue à réduire *in vitro* et *in vivo* une inflammation gastro-intestinale, en particulier une inflammation intestinale, chez un individu. Comme illustré dans les exemples et décrit ci-après, les inventeurs ont démontré que, par contre, la souche de *F. prausnitzii* I-4575 ne possède pas de telles propriétés avantageuses.

La souche selon l'invention est plus particulièrement capable de réduire la production de molécules pro-inflammatoires, telles que l'interleukine 8 (IL-8), l'interleukine 2 (IL-2), l'interleukine 4 (IL-4), l'interleukine 6 (IL-6) et l'interféron gamma (INFγ), et augmenter la production de molécules anti-inflammatoires, telles que l'interleukine 10 (IL-10).

Selon un premier objet, la présente invention concerne une souche bactérienne de l'espèce *Faecalibacterium prausnitzii* déposée auprès de la CNCM sous le numéro d'accession CNCM I-4573, pour son utilisation dans le traitement et/ou la prévention d'une maladie gastro-intestinale inflammatoire chez un individu, en particulier d'une affection intestinale inflammatoire chez un individu, plus particulièrement d'une affection intestinale inflammatoire colique chez un individu.

Cette souche identifiée par les inventeurs est donc une souche probiotique qui peut être utilisée pour les applications indiquées ci-dessus.

Un individu selon l'invention est de préférence un mammifère, y compris un mammifère non humain, et est, en particulier, un humain.

La maladie gastro-intestinale inflammatoire est, en particulier, une affection intestinale inflammatoire (IBD), plus particulièrement une affection intestinale inflammatoire colique.

Ladite affection intestinale inflammatoire colique peut, en particulier, être choisie dans le groupe constitué de la maladie de Crohn, la recto-colite hémorragique et la pochite, en particulier la maladie de Crohn et la recto-colite hémorragique.

Selon un autre mode de réalisation, la souche bactérienne pour son utilisation selon l'invention est comprise dans une composition comprenant un milieu physiologiquement acceptable, de préférence dans une composition orale, et de manière plus particulièrement préférée dans un supplément alimentaire.

Les termes « *milieu physiologiquement acceptable* » sont destinés à désigner un milieu qui est compatible avec l'organisme de l'individu auquel ladite composition doit être administrée. Il peut s'agir, par exemple, d'un solvant non toxique tel que l'eau. En particulier, ledit milieu est compatible avec une administration orale.

Une composition de l'invention est de préférence pour la voie orale.

Une composition de l'invention pour la voie orale peut être choisie dans le groupe constitué d'un produit alimentaire, une boisson, un produit pharmaceutique, un nutraceutique, un additif alimentaire, un supplément alimentaire et un produit laitier et est, en particulier, sous la forme d'un supplément alimentaire.

### Légendes des figures

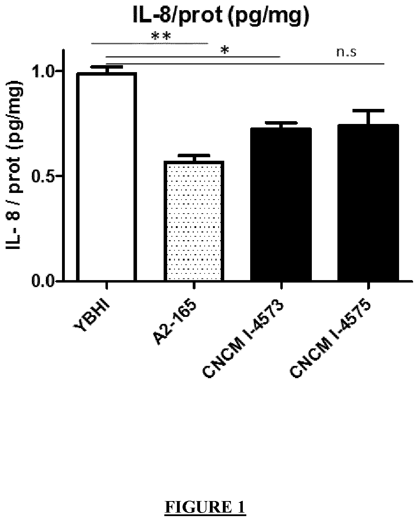

La figure 1 illustre la concentration d'interleukine 8 (IL-8) (en pg de IL-8/mg de protéines) dans des cellules Ht-29 stimulées par TNFα : (a) uniquement dans du milieu YBHI (témoin) ; (b) traitées avec la souche A2-165 de *F. prausnitzii ;* (c) traitées avec la souche 1-4573 de *F. prausnitzii* selon l'invention ; ou (d) traitées avec la souche 1-4575 de *F. prausnitzii.* Les essais sont conduits en triple. Les résultats sont normalisés en utilisant, en tant que valeur de référence, IL-8 produit après la co-induction avec PBS en tant que témoin négatif. *p<0,05.

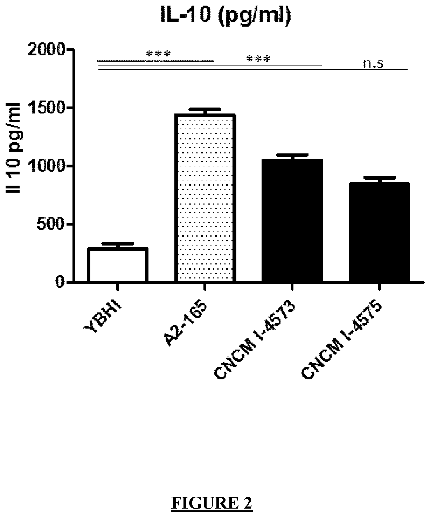

La figure 2 illustre la concentration d'interleukine 10 (IL-10) (en pg de IL-10/ml de protéines) dans des cellules mononucléaires de sang périphérique (PBMC) : (a) uniquement dans du milieu YBHI (témoin); (b) traitées avec la souche A2-165 de *F. prausnitzii ;* (c) traitées avec la souche 1-4573 de *F. prausnitzii* selon l'invention ; ou (d) traitées avec la souche I-4575 de *F. prausnitzii.* Les essais sont effectués en triple. ***p<0,001.

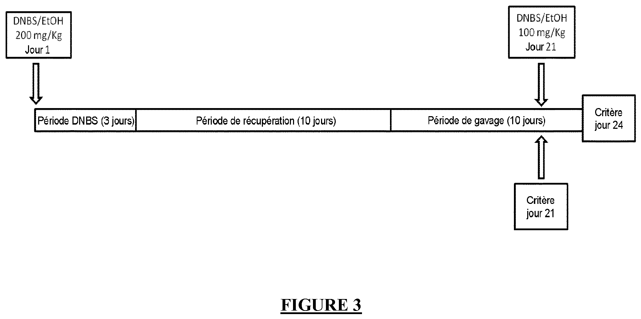

La figure 3 illustre le protocole expérimental pour le modèle de colite chronique chez la souris (DNBS).

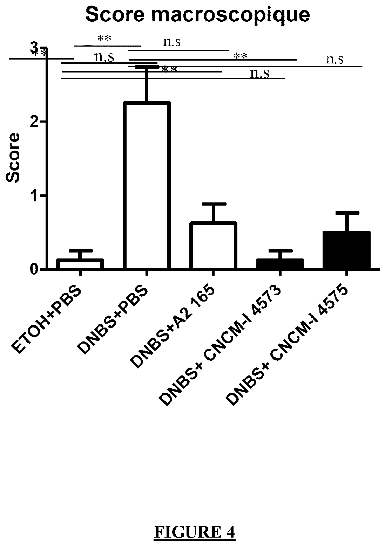

La figure 4 illustre les scores macroscopiques du côlon et de l'intestin grêle (a) de souris témoins (sans colite) recevant uniquement EtOH ; (b) de souris témoins présentant une colite DNBS et traitées avec PBS ; (c) de souris présentant une colite DNBS et traitées avec la souche A2-165 de *F. prausnitzii* ; (d) de souris présentant une colite DNBS et traitées avec la souche I-4573 de *F. prausnitzii* ; et (e) de souris présentant une colite DNBS et traitées avec la souche I-4575 de *F. prausnitzii.* Les critères macroscopiques (évalués sur une échelle de 0 (sans dommage) à 9) comprennent les dommages macroscopiques des muqueuses (tels que les ulcères, un épaississement de la paroi du côlon, la présence d'adhésions entre le côlon et d'autres organes intra-abdominaux), la consistance des matières fécales (en tant qu'indicateur de diarrhée) et la présence d'hyperémie. n=8. **p<0,01.

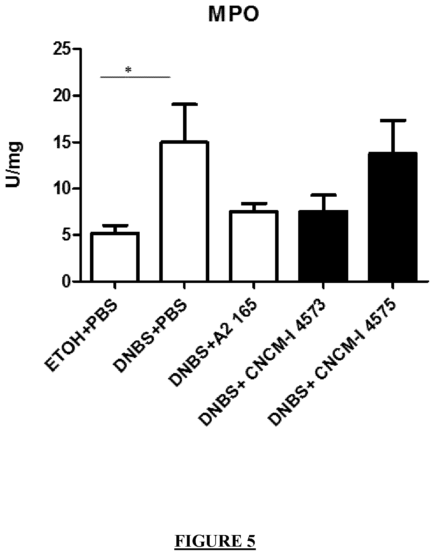

La figure 5 illustre l'activité myéloperoxydase (MPO) (U/mg) dans le côlon distal (a) de souris témoins (sans colite) recevant uniquement EtOH ; (b) de souris témoins présentant une colite DNBS et traitées avec PBS ; (c) de souris présentant une colite DNBS et traitées avec la souche A2-165 de *F. prausnitzii* ; (d) de souris présentant une colite DNBS et traitées avec la souche I-4573 de *F. prausnitzii* ; et (e) de souris présentant une colite DNBS et traitées avec la souche I-4575 de *F. prausnitzii.* n=8. *p<0,05.

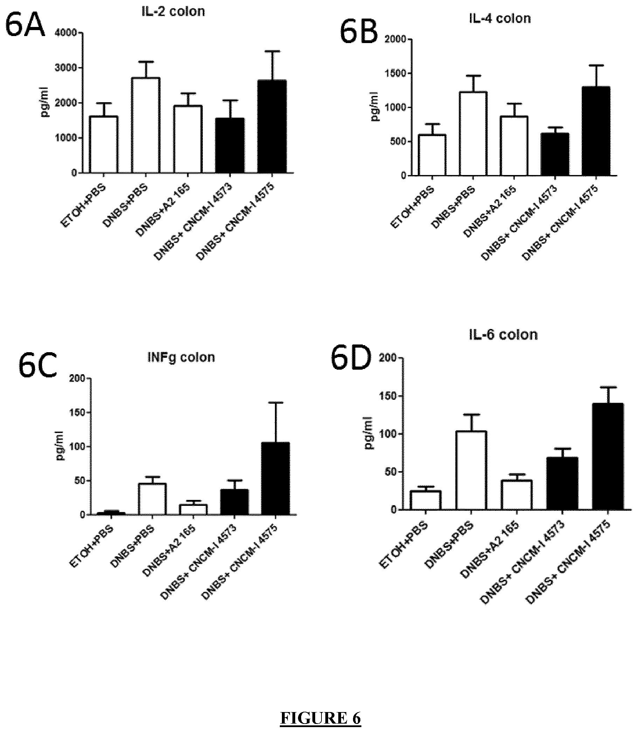

La figure 6 illustre les concentrations de cytokines coliques ((figure 6A) IL-2, (figure 6B) IL-4 (figure 6C) IL-6 et (figure 6D) IFNg) (en pg/ml) (a) de souris témoins (sans colite) recevant uniquement EtOH ; (b) de souris témoins présentant une colite DNBS et traitées avec PBS ; (c) de souris présentant colite DNBS et traitées avec la souche A2-165 de *F. prausnitzii* ; (d) de souris présentant une colite DNBS et traitées avec la souche I-4573 de *F. prausnitzii* ; et (e) de souris présentant une colite DNBS et traitées avec la souche I-4575 de *F. prausnitzii.*

### Description détaillée de l'invention

Les présents inventeurs ont conduit des travaux approfondis afin d'identifier la capacité d'une souche spécifique de *Faecalibacterium prausnitzii* à traiter et/ou prévenir des maladies gastro-intestinales inflammatoires chez un individu, en particulier des affections intestinales inflammatoires, chez un individu.

En effet, les inventeurs ont déterminé de façon inattendue que la souche de *F. prausnitzii* I-4573 possède la capacité à réduire une inflammation gastro-intestinale, en particulier une inflammation intestinale, chez un individu.

Il est présentement décrit que la souche bactérienne de l'invention peut, en particulier, réduire la concentration de molécules pro-inflammatoires, telles que l'interleukine 8 (IL-8), l'interleukine 2 (IL-2), l'interleukine 4 (IL-4) et l'interleukine 6 (IL-6).

Il est également présentement démontré que la souche bactérienne de l'invention est, en particulier, capable d'augmenter la concentration de molécules anti-inflammatoires, telles que l'interleukine 10 (IL-10).

### Souche de Faecalibacterium prausnitzii de l'invention

*F. prausnitzii* est un membre majeur du phylum *Firmicutes* et fait partie des bactéries commensales les plus abondants dans le microbiote du gros intestin sain humain.

*F. prausnitzii* est une bactérie extrêmement sensible l'oxygène (EOS) et est donc difficile à cultiver, même dans des conditions anaérobies (Duncan et al. 2002, Int. J. Syst. Evol. Microbiol. 52(Pt 6): 2141-6 et Lopez-Siles et al. Appl. Environ Microbiol. Janvier 2012 ; 78 (2):420-8). *F. prausnitzii* est, en particulier, connu comme étant l'une des bactéries productrices de butyrate les plus abondantes dans le tube digestif humain, l'acide gras à chaîne courte butyrate étant très important dans la physiologie de l'intestin, des fonctions systémiques et des effets bénéfiques pour la santé humaine (Macfarlane et Macfarlane (2011), J. Clin. Gastroenterol. 45 Suppl: S120-7).

La souche de *F. prausnitzii* A2-165 est également connu pour avoir des effets anti-inflammatoires et protecteurs dans des modèles murins de colite aiguë et chronique, c'est-à-dire dans des troubles inflammatoires (Martin et al., Inflamm Bowel Dis. Mars 2014 ; 20(3):417-30 et Sokol et al., Proc Natl Acad Sci USA. 28 octobre 2008 ; 105(43): 16731-6).

Les propriétés anti-inflammatoires de la souche A2-165 ne peuvent généralement pas être attribuées à l'espèce *F. prausnitz,* étant donné que l'existence de propriétés anti-inflammatoires est imprévisible pour une souche donnée de *F. prausnitzii.* En effet, une telle activité anti-inflammatoire spécifique est illustrée dans les exemples, dans lesquels un essai comparatif a été conduit avec une souche de *F. prausnitzii* ne faisant pas partie de l'invention, à savoir la souche CNCM I-4575, qui ne possède pas l'activité anti-inflammatoire de la souche de l'invention.

Une dose journalière adaptée d'une souche bactérienne selon l'invention est de 10⁷ à 10¹¹ unités formant des colonies (ufc) en tant que médicament, par exemple sous la forme d'une dose journalière équivalente à 10⁹ ufc.

Une souche de *Faecalibacterium prausnitzii* de l'invention est pour son utilisation dans le traitement et/ou la prévention d'une inflammation gastro-intestinale chez un individu, en particulier d'une inflammation de l'intestin chez un individu.

La souche de l'invention est un probiotique dont l'activité est située dans l'intestin. Une bactérie probiotique selon l'invention désigne une bactérie qui, lorsqu'elle est ingérée vivante en quantités suffisantes, peut exercer des effets bénéfiques sur la santé humaine.

Par conséquent, cette souche doit être administrée vivante à l'intestin.

La souche bactérienne de l'invention peut être administrée à l'intestin d'un individu à traiter de différentes façons, à savoir par voie orale ou rectale. Une bactérie selon l'invention est, de préférence, administrée par la voie orale.

Selon un mode de réalisation préféré, la souche bactérienne de l'invention est comprise dans une composition comprenant un milieu physiologiquement acceptable. Une telle composition est, de préférence, pour la voie orale, et en particulier sous la forme d'un supplément alimentaire.

### Compositions

La présente invention concerne en outre une composition comprenant, dans un milieu physiologiquement acceptable, au moins la souche bactérienne de *Faecalibacterium prausnitzii* I-4573 de l'invention.

Une composition selon l'invention est prévue pour le tube digestif, en particulier l'intestin.

Par conséquent, une composition selon l'invention est choisie parmi une composition orale ou rectale. Une composition de l'invention est, de préférence, une composition orale ou rectale, plus préférablement une composition orale.

Selon un mode de réalisation, une composition de l'invention est une composition orale, c'est-à-dire qu'elle est prévue pour une administration orale à un sujet.

Une telle composition peut être sous la forme d'une suspension, un comprimé, une pilule, une capsule, un granulé ou une poudre.

La composition selon l'invention pour la voie orale peut être choisie dans le groupe constitué d'un produit alimentaire, une boisson, un produit pharmaceutique, un nutraceutique, un additif alimentaire, un supplément alimentaire ou un produit laitier, et est, en particulier, un supplément alimentaire.

Selon un mode de réalisation préféré, une composition selon l'invention est un supplément alimentaire.

Un supplément alimentaire pour une administration orale peut être présent dans des capsules, des gélules, des capsules molles, des comprimés, des comprimés dragéifiés, des pilules, des pâtes, des pastilles, des gommes, des solutions ou émulsions buvables, un sirop ou un gel.

Avantageusement, une composition selon l'invention, prévue pour une administration orale, peut être pourvu d'un enrobage résistant au suc gastrique, afin d'assurer que la souche bactérienne de l'invention comprise dans ladite composition puisse traverser l'estomac endommagé. La libération de la souche bactérienne peut ainsi se produire pour la première fois dans le tractus intestinal supérieur.

Un supplément alimentaire selon l'invention peut comprendre en outre un édulcorant, un stabilisant, un antioxydant, un additif, un agent aromatisant et/ou un colorant.

La formulation de celui-ci est effectuée au moyen des procédés usuels pour produire des comprimés dragéifiés, des gélules, des gels, des hydrogels pour libération contrôlée, des émulsions, des comprimés ou des capsules.

Une composition selon l'invention peut également être sous la forme d'une composition nutritionnelle.

Une composition nutritionnelle selon l'invention est sous la forme d'un yogourt, une barre de céréale, des céréales pour le petit-déjeuner, un dessert, un aliment congelé, une soupe, un aliment pour animaux de compagnie, une suspension liquide, une poudre, un comprimé, une gomme ou un bonbon.

Dans un autre mode de réalisation de l'invention, une composition contenant la souche bactérienne de l'invention est administrée par voie intrarectale.

De préférence, une administration rectale est conduite sous la forme d'un suppositoire, un lavement ou une mousse.

En particulier, une composition de l'invention est adaptée pour l'administration d'une dose journalière représentant de 10⁷ à 10¹¹ unités formant des colonies (ufc) en tant que médicament, de préférence une dose journalière équivalente à 10⁹ ufc.

Par exemple, une composition selon l'invention peut être administrée à un individu en ayant besoin à une dose journalière unique de 1 g contenant la souche bactérienne I-4573 de l'invention en une quantité équivalente à une dose comprise entre 10⁷ et 10¹¹ ufc, de préférence 10⁹ ufc.

Dans un autre exemple, une composition selon l'invention peut être administrée à un individu en ayant besoin à une dose journalière unique de 0,2 g contenant la souche bactérienne I-4573 de l'invention en une quantité équivalente à une quantité comprise entre 10⁷ et 10¹¹ ufc, de préférence 10⁹ ufc.

Dans un autre exemple, une composition selon l'invention peut être administrée à un individu en ayant besoin deux fois par jour sur la base de deux doses de 1 g, chaque dose contenant, indépendamment, la souche bactérienne I-4573 de l'invention en une quantité équivalente à une quantité comprise entre 5.10⁶ et 5.10¹⁰ ufc (sur la base du poids sec), de préférence 5.10⁸ ufc, de sorte que la dose journalière totale de souche bactérienne I-4573 de l'invention administrée à l'individu soit telle qu'indiquée ci-dessus.

Une composition selon l'invention peut comprendre en outre au moins l'un parmi : des antioxydants, des huiles de poisson, DHA, EPA, des vitamines, des minéraux, des phytonutriments, une protéine, un lipide, des probiotiques et des combinaisons de ceux-ci.

L'invention est décrite ci-dessous de façon plus détaillée au moyen des exemples suivants qui sont présentés à titre d'illustration uniquement.

Toutes les références à des pourcentages sont des pourcentages en poids sauf indication contraire.

### EXEMPLES

La souche de *F. prausnitzii* selon l'invention déposée auprès de la CNCM sous le numéro d'accession CNCM I-4573 a été testée et comparée à deux autres souches de *F. prausnitzii,* A2-165 et CNCM I-4575, pour sa capacité à moduler *in vitro* et *in vivo* la réponse immunitaire et à avoir un impact direct sur l'inflammation intestinale.

Dans les essais *in vitro,* deux modèles cellulaires différents sont utilisés : HT-29 et PBMC.

Dans les essais *in vivo,* un modèle de colite murine est utilisé.

### Essais in vitro

### I. Souches bactériennes et conditions de culture

Les isolats des souches de *F. prausnitzii* testées sont cultivés dans du milieu YBHI (milieu de perfusion cerveau-cœur supplémenté avec 0,5 % d'extrait de levure) (Difco, Detroit, États-Unis) supplémenté avec 1 mg/ml de cellobiose (Sigma-Aldrich Chemie Gmbh, Buchs, Suisse), 1 mg/ml de maltose (Sigma-Aldrich) et 0,5 mg/ml de cystéine (Sigma-Aldrich) à 37 °C dans une chambre anaérobie remplie de N₂=85 %, CO₂=10 % et H₂=5 %.

Les souches sont isolées à partir de patients sains :
- souches CNCM-I4573 et CNCM-I4575 à partir d'un homme âgé de 40 ans ; et
- souche A2-165 (DSMZ 17677) à partir d'une femme âgée de 34 ans de Duncan SH et al. IJSEM, 2002, 52(Pt 6):2141-2146.

### II. Propriétés immunomodulatrices au moyen de cellules HT-29

### A. Culture de cellules

La lignée cellulaire HT-29 (ATCC HTB-38) (LGC-Standars) est cultivée dans du milieu essentiel minimal Eagle modifié par Dulbecco (DMEM) (Sigma-Aldrich) supplémenté avec 10 % (m/v) de sérum fœtal bovin (FBS) inactivé par la chaleur (GibcoBRL, Eragny, France) et avec pénicilline G/ streptomycine (5 000 UI/ml, 5 000 µg/ml) (Sigma-Aldrich). Les cultures sont incubées dans des fioles de culture de tissu de 25 cm² (Nunc, Roskilde, Danemark) à 37 °C dans une atmosphère à 5% (v/v) CO₂ jusqu'à confluence.

### B. Essais anti-inflammatoires

Des essais anti-inflammatoires utilisant HT-29 sont conduits selon la procédure décrite par Kechaou et al. (Applied and environmental microbiology 2012, 79(5):1491-1499).

Brièvement, 50 000 cellules HT-29 par puits sont ensemencées dans des plaques de culture à 24 puits (Nunc). Vingt-quatre heures avant stimulation bactérienne, le milieu de culture est remplacé par un milieu avec 5 % FBS.

Les essais commencent au jour 7 après ensemencement, lorsque les cellules sont à confluence (1,83X10⁶ cellules/puits). Vingt-quatre heures avant la co-culture bactérienne (jour 6), le milieu de culture est remplacé par un milieu avec 5 % FBS inactivé par la chaleur et 1 % glutamine.

Le jour de la co-culture, 10 % du surnageant bactérien est ajouté dans du DMEM dans un volume total de 500 µl. Les cellules sont stimulées simultanément avec TNF-α humain (5 ng/ml ; Peprotech, NJ) pendant 6 h à 37 °C dans 10 % CO₂.

Tous les échantillons sont analysés en triple.

Après co-incubation, les surnageants de cellules sont collectés et conservés à -80 °C jusqu'à analyse ultérieure des concentrations d'interleukine 8 (IL-8) par ELISA (Biolegend, San Diego, CA).

Les protéines totales sont déterminées au moyen du test du réactif de Bradford (Sigma-Aldrich). Les essais sont conduits au moins en triple.

Les résultats sont exprimés en IL-8/protéine (pg/mg) et sont normalisés en utilisant, en tant que valeur de référence, IL-8 produite après la co-incubation avec PBS en tant que témoin négatif.

### C. Analyse statistique

L'analyse statistique est effectuée au moyen du logiciel GraphPad (GraphPad Software, La Jolla, CA, États-Unis). Toutes les données sont exprimées sous forme de moyenne +/- SEM.

Les comparaisons sont réalisées avec le test de Kruskal-Wallis non paramétrique suivi par un test de comparaison multiple de Dunn.

Les tests de corrélation sont effectués au moyen du test de Spearman.

Une valeur *p* inférieure à 0,05 est considérée comme étant significative.

### D. Résultats

Les résultats obtenus sont présentés sur la figure 1.

Il peut être observé à partir de ces essais que la souche CNCM I-4573 est capable de réduire significativement la production de cytokine pro-inflammatoire IL-8 induite par la stimulation de TNF-α dans des cellules épithéliales HT-29.

Comme prévu, la souche de *F. prausnitzii* A2-165 produit également une telle réduction de la production de IL-8.

Au contraire, la souche de *F. prausnitzii* I-4575 n'induit pas une diminution statistiquement significative de IL-8.

### III. Propriétés immunomodulatrices au moyen de PBMC

### A. Culture de cellules

Des PBMC commerciaux (StemCell Technologies, France) de cinq donneurs sains sont utilisés.

Les donneurs présentent les caractéristiques suivantes : hommes, âgés de moins de 65 ans, indice de masse corporelle < 30, non-fumeur, pas de médicaments ayant des effets anti-inflammatoires connus pris au cours des 15 jours précédents la sélection, et test négatif pour les virus VIH, hépatite A et hépatite B.

Après réception, les cellules sont conservées dans de l'azote liquide jusqu'à utilisation.

### B. Essais anti-inflammatoires

Afin de préparer des PBMC pour des essais de co-culture avec des bactéries *F. prausnitzii,* les flacons sont décongelés à 37 °C dans un bain-marie et ensuite transféré dans un milieu contenant du milieu RPMI-1640 supplémenté avec 10 % FCS inactivé par la chaleur, 1 % L-glutamine et 0,1 % pénicilline/streptavidine (les composants du milieu sont achetés à Lonza, Suisse). DNase (100 µg/ml, Roche Applied Science, France) est ajouté au mélange afin d'éviter l'agglomération des cellules.

Les cellules sont ensuite centrifugées à 200 g pendant 15 min, comptées en utilisant du bleu trypan et étalées sur des plaques de 24 puits à 1X10⁶ cellules/puits. Des surnageants sont ajoutés en triple (trois puits par donneur) à 10 % dans un volume total de 1 ml. Les plaques sont incubées pendant 24 h à 37 °C avec 10 % CO₂.

Les surnageants de culture sont collectés, mélangés avec un cocktail d'antiprotéases conformément aux instructions du fabricant (EDTA-inhibiteur de protéase complet, Roche Applied Bioscience) et conservés à -80 °C jusqu'à analyse ultérieure des concentrations d'interleukine 10 (IL-10) par ELISA (Mabtech, Suède).

### C. Analyse statistique

L'analyse statistique est effectuée au moyen du logiciel GraphPad (GraphPad Software, La Jolla, CA, États-Unis). Toutes les données sont exprimées sous forme de moyenne +/- SEM.

Les comparaisons sont réalisées avec le test de Kruskal-Wallis non paramétrique suivi par un test de comparaison multiple de Dunn.

Les tests de corrélation sont effectués au moyen du test de Spearman.

Une valeur p inférieure à 0,05 est considérée comme étant significative.

### D. Résultats

Les résultats obtenus sont présentés sur la figure 2.

Ces essais démontrent que la souche CNCM I-4573 est capable d'augmenter significativement la production de cytokine anti-inflammatoire IL-10 dans des PBMC.

Comme prévu, la souche de *F. prausnitzii* A2-165 produit également une telle augmentation de la production de IL-10.

Au contraire, la souche de *F. prausnitzii* I-4575 n'induit pas une augmentation statistiquement significative de IL-10.

### Essais in vivo

### A. Induction de colite DNBS et administration de bactéries

Le protocole de colite induite par DNBS comme précédemment décrit (Martin R, et al. Inflamm Bowel Dis. Mars 2014 ; 20(3):417-30).

Brièvement, les souris sont anesthésiées avec de l'enflurane (Abbott, Abbott Park, IL) et un segment de 10 cm de longueur de tubulure PE-90 (ClayAdam, Parsippany, NJ) est fixé à une seringue de tuberculine et inséré à 3,5 cm dans le côlon.

La colite est induite par injection intrarectale (i.r.) par l'intermédiaire de ce tube de 200 mg/kg de solution de DiNitroBenzène-acide Sulfonique (DNBS) (ICN, Biomedical Inc.) dans de l'éthanol à 30 % (EtOH).

Les souris témoins (sans colite) reçoivent uniquement EtOH.

Les souris sont alimentées avec 6 % saccharose dans de l'eau de boisson pendant 3 premiers jours après l'injection de DNBS pour prévenir la déshydratation (période DNBS). 10 jours après la période DNBS, 200 µl contenant 1X10⁹ UFC d'une des souches bactériennes sont administrées par voie intragastrique, chaque jour pendant 10 jours (période de gavage) (voir figure 3).

Les groupes d'étude sont comme suit : groupe témoin non-colite (EtOH + PBS), groupe témoin colite (DNBS + PBS), groupe de souche de *F. prausnitzii* A2-165 (DNBS + A2-165), *F. prausnitzii* CNCM-I4573 (DNBS + CNCM-I4573) et *F. prausnitzii* CNCM-I4575 (DNBS + CNCM-I4575).

La colite est réactivée 21 jours après la première injection de DNBS (période de récupération) avec une deuxième injection de 100 mg/kg de solution de DNBS.

La gravité de la colite est ensuite déterminée en surveillant la perte de poids (résultats non présentés) pendant les 3 jours suivant la deuxième injection.

### B. Scores macroscopiques

Les souris sont sacrifiées par dislocation cervicale et la cavité abdominale est ouverte. Le côlon et l'intestin grêle sont prélevés et ouverts longitudinalement ; les dommages macroscopiques sont évalués immédiatement.

Les scores macroscopiques sont enregistrés au moyen d'un système précédemment décrit pour la colite DNBS.

Brièvement, les critères macroscopiques (évalués sur une échelle de 0 à 9) comprennent les dommages macroscopiques des muqueuses (tels que les ulcères, un épaississement de la paroi du côlon, la présence d'adhésions entre le côlon et d'autres organes intra-abdominaux), la consistance des matières fécales (en tant qu'indicateur de diarrhée) et la présence d'hyperémie.

Les résultats obtenus sont représentés sur la figure 4.

### C. Activité myéloperoxydase (MPO)

L'activité myéloperoxydase (MPO), utilisée en tant que marqueur d'infiltration neutrophilique, est dosée par une version modifiée du procédé décrit par Bradley et al. (J. Invest. Dermatol. 1982, 78(3):206-209).

Une longueur d'un centimètre de côlon distal est récupérée et homogénéisée (50 mg/ml) dans du tampon phosphate de potassium 50 mM glacé (pH 6) contenant 5 % de bromure d'hexadécyltriméthylammonium (Sigma-Aldrich) et du peroxyde d'hydrogène (H₂O₂).

La réaction colorimétrique est suivie en mesurant l'absorbance avec un spectrophotomètre.

Les résultats obtenus sont représentés sur la figure 5.

### D. Dosages de cytokine

Une longueur d'un centimètre de côlon distal est récupérée et homogénéisée dans 400 µl de tampon Tris-HCl contenant des inhibiteurs de protéase (Sigma-Aldrich) dans un lyseur de tissu.

Les échantillons sont centrifugés pendant 20 min et le surnageant est congelé à -80 °C jusqu'à analyse ultérieure.

Avant le sacrifice, les échantillons de sang sont obtenus à partir du plexus veineux rétro-orbital et centrifugés, puis les sérums sont conservés à -80 °C jusqu'à analyse.

Les cytokines pro-inflammatoires (IL-6, IL-4, IFN-γ, et IL-2) sont dosées au moins d'un système de réseau de billes cytométriques (BD, NJ, États-Unis).

Les résultats obtenus sont présentés sur les figures 6a (IL-2), 6b (IL-4), 6c (IFN-γ) et 6d (IL-6).

### E. Analyse statistique

Le logiciel GraphPad (GraphPad Sofware, La Jolla, CA, États-Unis) est utilisé pour l'analyse statistique.

Les résultats sont présentés sous forme de graphiques à barres +/- SEM.

Les comparaisons sont réalisées avec le test de Kruskal-Wallis non paramétrique suivi par un test de comparaison multiple de Dunn. Les tests de corrélation sont effectués au moyen du test de Spearman.

Une valeur p inférieure à 0,05 est considérée comme étant significative.

### F. Résultats

**1.** Les scores macroscopiques de la figure 4 montrent qu'un très bon score est obtenu chez des souris DNBS traitées avec la souche I-4573, équivalent à celui obtenu chez les souris témoins ne présentant pas de colite.
   Au contraire, les scores obtenus avec des souris DNBS traitées avec la souche A2-165 ou la souche I-4575 sont plus élevés sur l'échelle des critères macroscopiques que ceux obtenus avec les souris témoins ne présentant pas de colite et les souris DNBS traitées avec la souche I-4573.
**2.** En ce qui concerne les activités myéloperoxydase mesurées, les activités obtenues avec des souris DNBS traitées avec la souche I-4573 ou avec la souche A2-165 sont pratiquement similaires à l'activité obtenue avec les souris témoins ne présentant pas de colite.
   Au contraire, l'activité obtenue avec des souris DNBS traitées avec la souche I-4575 est similaire à l'activité obtenue avec des souris DBNS témoins non traitées.
**3.** Finalement, le profil de cytokines pro-inflammatoires obtenu avec des souris DNBS traitées avec la souche I-4573 et des souris DNBS traitées avec la souche A2-165 démontre clairement l'effet anti-inflammatoire de ces deux souches.

En fait, en ce qui concerne, en particulier, les souris DNBS traitées avec la souche I-4573, les taux d'expression de IL-2 et IL-4 sont similaires à ceux obtenus avec des souris témoins ne présentant pas de colite. Les taux d'expression de IFN- γ et IL-6 sont plus réduits par rapport à ceux obtenus avec les souris DNBS témoins non traitées.

Au contraire, la souche I-4575 ne présente aucune propriété anti-inflammatoire. Ses taux d'expression de IL-2, IL-4, IL-6 et IFN-γ sont similaires, ou même supérieurs, à ceux observés avec les souris DNBS témoins non traitées.

En conséquence, il est démontré dans les présents exemples que la souche de *F. prausnitzii* I-7573 présente avantageusement des propriétés anti-inflammatoires et que toutes les souches de *F. prausnitzii* ne possèdent pas de telles propriétés, comme démontré avec la souche I-4575.

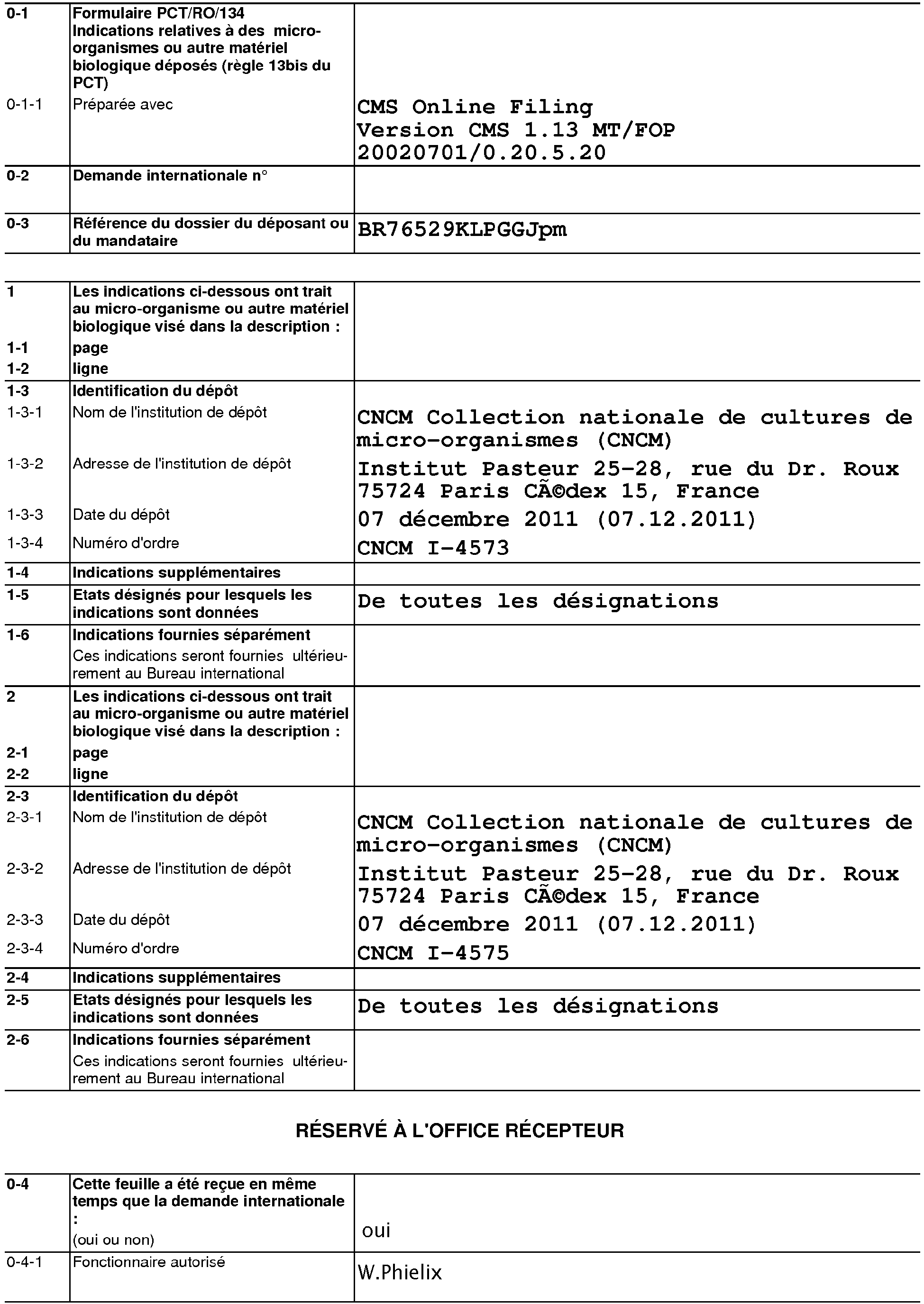

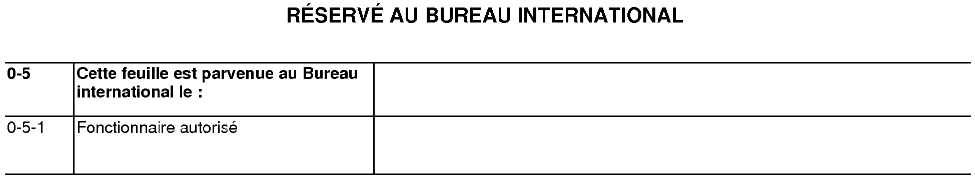

## Revendications

1. Souche bactérienne de l'espèce *Faecalibacterium prausnitzii* déposée auprès de la CNCM sous le numéro d'accession CNCM I-4573, pour son utilisation dans le traitement et/ou la prévention d'une maladie gastro-intestinale inflammatoire chez un individu.

2. Souche bactérienne pour son utilisation selon la revendication 1, **caractérisée en ce que** l'individu est un mammifère.

3. Souche bactérienne pour son utilisation selon la revendication 1 ou 2, **caractérisée en ce que** l'individu est un humain.

4. Souche bactérienne pour son utilisation selon l'une quelconque des revendications 1 à 3, **caractérisée en ce que** la maladie gastro-intestinale inflammatoire est une affection intestinale inflammatoire.

5. Souche bactérienne pour son utilisation selon l'une quelconque des revendications précédentes, **caractérisée en ce que** la maladie gastro-intestinale inflammatoire est une affection intestinale inflammatoire colique.

6. Souche bactérienne pour son utilisation selon la revendication 5, **caractérisée en ce que** l'affection intestinale inflammatoire colique est choisie dans le groupe constitué de la maladie de Crohn, la recto-colite hémorragique et la pochite.

7. Souche bactérienne pour son utilisation selon la revendication 5 ou 6, **caractérisée en ce que** l'affection intestinale inflammatoire colique est choisie dans le groupe constitué de la maladie de Crohn et la recto-colite hémorragique.

8. Souche bactérienne pour son utilisation selon l'une quelconque des revendications précédentes, **caractérisée en ce que** la souche bactérienne est comprise dans une composition comprenant un milieu physiologiquement acceptable.

9. Souche bactérienne pour son utilisation selon l'une quelconque des revendications précédentes, **caractérisée en ce que** la souche bactérienne est comprise dans une composition orale.

10. Souche bactérienne pour son utilisation selon l'une quelconque des revendications précédentes, **caractérisée en ce que** la souche bactérienne est comprise dans un supplément alimentaire.

11. Souche bactérienne pour son utilisation selon l'une quelconque des revendications 8 à 10, **caractérisée en ce que** la composition est adaptée pour l'administration d'une dose journalière représentant de 10⁷ à 10¹¹ unités formant des colonies (ufc) en tant que médicament, de préférence sous la forme d'une dose journalière équivalente à 10⁹ ufc.

12. Souche bactérienne pour son utilisation selon l'une quelconque des revendications 8 à 11, **caractérisée en ce que** la composition est pour la voie orale et est choisie dans le groupe constitué d'un produit alimentaire, une boisson, un produit pharmaceutique, un nutraceutique, un additif alimentaire, un supplément alimentaire et un produit laitier.

13. Souche bactérienne pour son utilisation selon la revendication 12, **caractérisée en ce que** la composition est pour la voie orale et est un supplément alimentaire.

## Patentansprüche

1. Bakterienstamm der Spezies *Faecalibacterium prausnitzii,* der bei der CNCM unter der Zugangsnummer CNCM I-4573 hinterlegt ist, zur Verwendung bei der Behandlung und/oder Vorbeugung einer entzündlichen Magen-Darm-Erkrankung bei einem Individuum.

2. Bakterienstamm zur Verwendung nach Anspruch 1, **dadurch gekennzeichnet, dass** das Individuum ein Säuger ist.

3. Bakterienstamm zur Verwendung nach Anspruch 1 oder 2, **dadurch gekennzeichnet, dass** das Individuum ein Mensch ist.

4. Bakterienstamm zur Verwendung nach einem der Ansprüche 1 bis 3, **dadurch gekennzeichnet, dass** die entzündliche Magen-Darm-Erkrankung eine entzündliche Darmkrankheit ist.

5. Bakterienstamm zur Verwendung nach einem der vorhergehenden Ansprüche, **dadurch gekennzeichnet, dass** die entzündliche Magen-Darm-Erkrankung eine entzündliche Darmkrankheit des Kolons ist.

6. Bakterienstamm zur Verwendung nach Anspruch 5, **dadurch gekennzeichnet, dass** die entzündliche Darmkrankheit des Kolons aus der Gruppe bestehend aus Morbus Crohn, Colitis ulcerosa und Pouchitis ausgewählt ist.

7. Bakterienstamm zur Verwendung nach Anspruch 5 oder 6, **dadurch gekennzeichnet, dass** die entzündliche Darmkrankheit des Kolons aus der Gruppe bestehend aus Morbus Crohn und Colitis ulcerosa ausgewählt ist.

8. Bakterienstamm zur Verwendung nach einem der vorhergehenden Ansprüche, **dadurch gekennzeichnet, dass** der Bakterienstamm in einer Zusammensetzung enthalten ist, die ein physiologisch annehmbares Medium umfasst.

9. Bakterienstamm zur Verwendung nach einem der vorhergehenden Ansprüche, **dadurch gekennzeichnet, dass** der Bakterienstamm in einer oralen Zusammensetzung enthalten ist.

10. Bakterienstamm zur Verwendung nach einem der vorhergehenden Ansprüche, **dadurch gekennzeichnet, dass** der Bakterienstamm in einem Nahrungsergänzungsmittel enthalten ist.

11. Bakterienstamm zur Verwendung nach einem der Ansprüche 8 bis 10, **dadurch gekennzeichnet, dass** die Zusammensetzung für die Verabreichung einer Tagesdosis, die 10⁷ bis 10¹¹ koloniebildende Einheiten (KBE) darstellt, als Medikament, vorzugsweise in Form einer Tagesdosis entsprechend 10⁹ KBE, ausgelegt ist.

12. Bakterienstamm zur Verwendung nach einem der Ansprüche 8 bis 11, **dadurch gekennzeichnet, dass** die Zusammensetzung für den oralen Verabreichungsweg bestimmt ist und aus der Gruppe bestehend aus einem Lebensmittelprodukt, einem Getränk, einem pharmazeutischen Produkt, einem Nutrazeutikum, einem Lebensmittelzusatzstoff, einem Nahrungsergänzungsmittel und einem Milchprodukt ausgewählt ist.

13. Bakterienstamm zur Verwendung nach Anspruch 12, **dadurch gekennzeichnet, dass** die Zusammensetzung für den oralen Verabreichungsweg bestimmt und ein Nahrungsergänzungsmittel ist.

## Claims

1. Bacterial strain of the *Faecalibacterium prausnitzii* species deposited with the CNCM under accession number CNCM 1-4573, for use thereof in the treatment and/or prevention of an inflammatory gastrointestinal disease in an individual.

2. Bacterial strain for use thereof according to Claim 1, **characterized in that** the individual is a mammal.

3. Bacterial strain for use thereof according to Claim 1 or 2, **characterized in that** the individual is a human being.

4. Bacterial strain for use thereof according to any one of Claims 1 to 3, **characterized in that** the inflammatory gastrointestinal disease is an inflammatory bowel disorder.

5. Bacterial strain for use thereof according to any one of the preceding claims, **characterized in that** the inflammatory gastrointestinal disease is an inflammatory bowel disorder of the colon.

6. Bacterial strain for use thereof according to Claim 5, **characterized in that** the inflammatory bowel disease of the colon is chosen from the group consisting of Crohn's disease, ulcerative colitis and pouchitis.

7. Bacterial strain for use thereof according to Claim 5 or 6, **characterized in that** the inflammatory bowel disorder of the colon is chosen from the group consisting of Crohn's disease and ulcerative colitis.

8. Bacterial strain for use thereof according to any one of the preceding claims, **characterized in that** the bacterial strain is included in a composition comprising a physiologically acceptable medium.

9. Bacterial strain for use thereof according to any one of the preceding claims, **characterized in that** the bacterial strain is included in an oral composition.

10. Bacterial strain for use thereof according to any one of the preceding claims, **characterized in that** the bacterial strain is included in a food supplement.

11. Bacterial strain for use thereof according to any one of Claims 8 to 10, **characterized in that** the composition is suitable for the administration of a daily dose representing from 10⁷ to 10¹¹ colony-forming units (cfu) as medicine, preferably in the form of a daily dose equivalent to 10⁹ cfu.

12. Bacterial strain for use thereof according to any one of Claims 8 to 11, **characterized in that** the composition is for oral administration and is chosen from the group consisting of a food product, a drink, a pharmaceutical product, a nutraceutical, a food additive, a food supplement and a milk product.

13. Bacterial strain for use thereof according to Claim 12, **characterized in that** the composition is for oral administration and is a food supplement.
